Europäisches Patentamt

European Patent Office

Office européen des brevets

(11) Veröffentlichungsnummer: **0 483 655 A2**

# (12) EUROPÄISCHE PATENTANMELDUNG

(21) Anmeldenummer: **91118043.8**

(22) Anmeldetag: **23.10.91**

(51) Int. Cl.⁵: **C12N 1/14**, A01G 1/04

(30) Priorität: **31.10.90 DE 4034622**

(43) Veröffentlichungstag der Anmeldung:
**06.05.92 Patentblatt 92/19**

(84) Benannte Vertragsstaaten:
**AT BE CH DE DK ES FR GB GR IT LI LU NL SE**

(71) Anmelder: **Vogel, Ullrich, Dipl.-Ing.**
**Falkenweg 11**
**W-6272 Niedernhausen-Engenhahn(DE)**

(72) Erfinder: **Vogel, Ullrich, Dipl.-Ing.**
**Falkenweg 11**
**W-6272 Niedernhausen-Engenhahn(DE)**

(74) Vertreter: **Dr. Fuchs, Dr. Luderschmidt**
**Dipl.-Phys. Seids, Dr. Mehler Patentanwälte**
**Abraham-Lincoln-Strasse 7, Postfach 4660**
**W-6200 Wiesbaden(DE)**

(54) **Verfahren zum Kultivieren saprophytisch lebender Pilze.**

(57) Für die Massenproduktion saprophytisch lebender Pilze auf im wesentlichen lignozellulosehaltigem Substrat wird dieses unter Verzicht aufwendiger Pasteurisierungs- und Sterilisierungsverfahren bei 50-70°C in einem Zeitraum von vorzugsweise nur 1-3 Minuten konditioniert. Das mit Pilzbrutsubstanz inokulierte Substrat wird dann in der vegetativen Wachstumsphase bei einem Raumnutzungsgrad von mindestens 0,8 optimal mit Sauerstoff versorgt und die Stapelung des Substrats so vorgenommen, daß im Inneren des Substrats eine Temperatur von 30°C nicht überschritten wird. Der Raumnutzungsgrad in der generativen Wachstumsphase wird sodann auf 0,2-0,5 gesenkt. Dadurch wird eine gegenüber dem bisherigen Stand der Technik wesentliche Zeit-, Energie- und Raumeinsparung bei hohen Ausbeuten ermöglicht.

EP 0 483 655 A2

EP 0 483 655 A2

Die Erfindung betrifft ein Verfahren nach dem Oberbegriff des Anspruchs 1.

Verschiedene Verfahren zur Kultivierung von saprophytisch lebenden Pilzen sind bekannt. Den meisten Verfahren sind die folgenden, grundlegenden Schritte gemeinsam:

1. Konditionieren des Nährsubstrats,
2. Inokulation des konditionierten Nährsubstrats mit Pilzbrutsubstanz,
3. vegetative Wachstumsphase zur Myzelbildung,
4. generative Wachstumsphase zur Fruchtkörperbildung und
5. Erntephase.

Die größte Variationsbreite bei der Kultivierung findet sich im Hinblick auf Auswahl und Vorbehandlung des jeweiligen Nährsubstrats. Das Nährsubstrat enthält die für das Pilzwachstum erforderlichen Nährstoffe und Wasser. Die Vorbehandlung soll dazu dienen, die günstigsten Lebensbedingungen für das Wachstum der jeweiligen Pilzart bereitzustellen.

Wird unkonditioniertes Substrat mit Pilzbrut inokuliert, so können unerwünschte Bakterien und Fremdpilze im Nährsubstrat in Konkurrenz mit der Pilzkultur treten und deren Wachstum mehr oder weniger beeinträchtigen. Aus diesem Grund wird das Substrat einer Vorbehandlung unterzogen, wobei die Konkurrenzorganismen vor der Inokulation mit der Pilzbrut abgetötet werden oder ihr weiteres Wachstum unterbunden wird.

Eine Möglichkeit, die Konkurrenzorganismen aus dem Substrat zu eliminieren besteht darin, das Nährsubstrat zuerst in einem Autoklaven bei 100 bis 135°C zu sterilisieren und dann die Pilzbrut auf dem autoklavierten Substrat unter sterilen Bedingungen anwachsen zu lassen. Ein solches Verfahren ist wegen des apparativen Aufwandes und der zur Durchführung der Sterilisation benötigten Energie sehr kostenintensiv. Eine Verbesserung brachte das in der DE 1 632 919 beschriebene Verfahren, in dem unter Umgehung der sterilen Anwachsphase das autoklavierte, sterile Substrat vor der Inokulation und nach Abkühlen auf ca. 50°C mit vorkultivierten, spezifischen thermophilen Mikroorganismen beimpft und anschließend in einem gesteuerten und kontrollierten Fermentierungsprozeß zur Entwicklung gebracht wurde. Dabei wurde die Tatsache ausgenutzt, daß die Schutzbakterien katabole Ausscheidungsprodukte im Verlaufe des Fermentationsprozeßes bilden, die das Wachstum von eventuell während der unsterilen Anwachsphase eingeschleppten Konkurrenzorganismen hemmen können. Zur Aktivierung des Wachstums dieser thermophilen Mikroorganismen muß das Substrat aber bei Temperaturen von 50 bis 70°C gehalten werden, wobei der gesamte Fermentationsvorgang etwa 2 bis 5 Tage dauert.

Eine weitere Verbesserung brachte die einfache Temperaturbehandlung auf ca. 100°C ohne Überdruck (unter Vermeidung des Autoklavierens), wobei man auf eine totale Entkeimung des Substrats verzichtete und über diese Teilentkeimung (Pasteurisation) nur die vegetativen Formen der Mikroorganismen abtötet. Eine solche Teilentkeimung findet über einen Zeitraum von ca. 2 bis 5 Stunden statt, in dem immer noch ziemlich energieaufwendig ständig die zur Aufrechterhaltung dieser Temperatur erforderliche Energie zugeführt werden muß.

In der deutschen Patentschrift DE 2 125 692 wird auf die Entkeimung infolge Wärmebehandlung gänzlich verzichtet und das Wachstum der konkurrierenden Mikroorganismen nur noch mittels einer "wilden Fermentation" des Substrats unterdrückt. In Gegenwart thermophiler, aerorober Mikroorganismen wird das Substrat ca. 25 Stunden auf 60 bis 70°C erwärmt, während eines Zeitraums von 5 bis 10 Stunden sodann auf 45 bis 55°C abgekühlt und bei dieser Temperatur 3 Tage lang unter aeroben Bedingungen fermentieren gelassen. Ein ähnliches Fermentationsverfahren, jedoch unter im wesentlichen anaeroben Bedingungen, wird in der DE-OS 34 34 486 beschrieben, wobei erheblich kürzere Fermentationszeiten benötigt wurden (Erhitzen auf 60°C während einer Stunde und Fermentation bei 40 bis 45°C während 1 bis 2 Tagen).

In der DE-OS 24 50 183 schließlich wird unter Verzicht von sowohl thermischer Einwirkung als auch der Schutzwirkung thermophiler Mikroorganismen das Substrat direkt mit Benomyl (1-Butylcarbamoyl-2-Benzimidazolmethylcarbamat), einem Fungizid, behandelt. Nachdem aber Abbaustoffe davon (insbesondere Bendasamin) in den Fruchtkörpern der geernteten Pilze nachgewiesen werden konnten, konzentrierte sich das Hauptinteresse zur Substratkonditionierung wieder auf das "integrierte, mikrobiologische Verfahren", der Fermentation mittels thermophiler Schutzbakterien, wie sie in der DE 21 25 692 beschrieben wird.

Ein entschiedener Nachteil des Verfahrens liegt nach wie vor in der Zeitdauer, die für den Fermentationsprozeß benötigt wird. Die erforderliche ständige Überwachung der Temperatur und Aerobilität machen es erforderlich, daß das gesamte Substrat für einen Kultivierungszyklus in einem Arbeitsgang fermentiert werden muß, wofür dann bei größeren Chargen relativ großflächige Massenbehandlungsräume, sogenannte Feststoff-Fermentäre, benötigt werden. Der hierbei für die Vorfermentation des Substrats aufzuwendende Energiebedarf beträgt ca. 260 KW Stunden pro Tonne Substrat.

Nach der Bereitung des Substrats wird dieses mit Pilzbrutsubstanz beimpft. Dabei muß unter möglichst sterilen Bedingungen gearbeitet werden. Die Vermischung der Brutsubstanz mit dem vorfermentierten Substrat erfolgt in einem unmittelbar an die Feststoff-Fermentäre anschließenden möglichst steril zu haltenden Raum, der sogenannten Spickhalle, wofür bei der Massenproduktion von Pilzen gemäß der Menge des in einem Arbeitsgang vorfermentierten Substrats eine entsprechend große Grundfläche benötigt wird. Die technische Ausrüstung zur Aufrechterhaltung der erforderlichen Sterilität in solch großen Räumen ist entsprechend aufwendig.

Das mit Pilzbrut inokulierte Substrat wird sodann in Plastiksäcke, meistens aus Polyethylen, abgefüllt, die seitlich zum Herauswachsen der Fruchtkörper spezielle Perforationen aufweisen. Ein Plastiksack faßt etwa 25 bis 30 kg Substrat. Diese Plastiksäcke werden sodann, auf Metallrohre aufgespießt, in den Anwachshallen zur Ausbildung des vegetativen Myzelwachstums gespeichert. Nach einer Inkubationszeit von 21 Tagen werden sie sodann in die Erntehallen weitertransportiert, wobei zwischen den einzelnen in Gruppen zusammengefaßten Metallrohren, auf denen die Plastiksäcke aufgespießt sind, genügend breite Wege für die Pflückarbeiten vorgesehen sind.

Die für die Massenproduktion von Pilzen nach dem Stand der Technik gestellten Anforderungen an den Raumbedarf, basierend auf Feststoff-Fermentär, Spick-, Anwachs- und Erntehalle, sind relativ groß und im Hinblick auf die in den jeweiligen Räumen erwünschten klimatischen und sogar teilweise sterilen Bedingungen kosten- und energieintensiv. Der Erfindung liegt daher die Aufgabe zugrunde, ein Verfahren zur Verfügung zu stellen, das es gegenüber dem bisherigen Stand der Technik ermöglicht, saprophytisch lebende Pilze, insbesondere auch in Massenproduktion, zeit- energie- und raumsparend in hohen Ausbeuten zu erhalten.

Diese Aufgabe wird erfindungsgemäß dadurch gelöst, daß das Substrat unter Verzicht auch energie- und zeitaufwendige Pasteurisierungs- und Sterilisierungsverfahren in einem vorbereitenden Schritt konditioniert wird, nach Inokulation mit Pilzbrutsubstanz und Verdichten in festen, flachen stapelbaren Behältern im vegetativen Bereich und hoher Raumausnutzung einer optimalen Sauerstoff-Versorgung ausgesetzt wird und sodann im generativen Bereich unter im wesentlichen seitlicher Abstandvergrößerung der stapelbaren Behälter der Raumnutzungsgrad so gesenkt wird, daß bei gleichbleibender Luftdurchströmung, aber vermindertem $CO_2$-Partialdruck die sich bildenden Fruchtkörper auf der Substratoberfläche ungehindert in den Raum wachsen können.

Die stattfindende Feststofffermentation des Substrats durch den Kulturpilz erfogt dabei in mit verdichtetem inokuliertem Substrat beschickten, übereinander gestapelten Behältern, wobei zwischen nach oben freier Oberseite des einen Behälters und durchbrochenem Boden des darüber gestapelten Behälters ein Spalt bleibt, der von Luft durchströmt werden kann, welche einerseits für optimale Sauerstoffversorgung des inokulierten Substrates bzw. der sich darin entwickelnden Kulturpilzes und andererseits für die Abfuhr der sich entwickelnden Wärme sorgt. Die Höhe der einzelnen Behälter und damit die maximale Substratschichthöhe in den Behältern ist dabei so bemessen, daß der Wärmeaustausch zwischen Behälterinhalt und diesen Behälter überstreichender Luft im Zentrum (Kern) des im Behälter befindlichen inokulierten Substrats eine Temperatur von maximal 30°C aufrechterhalten kann. Der Grad des Wärmeaustauschs und damit die Substratschichthöhe in einem Behälter ist natürlich noch abhängig von der Wärmeleitfähigkeit des Substrats sowie vom Wärmeübergangskoeffizienten Substrat-Luft. Der Sauerstoffpartialdruck sollte dabei nicht unter 0,2 bar sinken (entsprechend einem $CO_2$-Partialdruck von ca. 0,005). Typische Behälterhöhen bei Verwendung von lignozellulosehaltigem Substrat liegen in der Größenordnung von 20 bis 25 cm. Der Abstand zwischen Oberseite des einen Behälters und Unterseite des darüber gestapelten Behälters beträgt typischerweise 2 bis 3 cm. Alternativ kann selbstverständlich auch ein aus dem Champignonanbau bekanntes Stellagensystem, insbesondere bei hohen Durchsatzraten, zur Anwendung kommen.

Unter Konditionierung wird in diesem Verfahren das wärmeerzeugende Zerkleinern des Rohstoffes zu einer Korngröße sowie das sich unmittelbar anschließende Tauchen in Wasser verstanden. Das Kurzzeittauchen findet bei Temperaturen zwischen 50 und 70°C, vorzugsweise bei 65°C über einen Zeitraum von 10 Sekunden bis 5 Minuten, vorzugsweise 1 bis 3 Minuten statt (Konti-Verfahren). Selbstverständlich kann das zerkleinerte Substrat auch bei niedrigeren Wassertemperaturen eingetaucht werden, der Rohstoff benötigt dann aber wegen der langsameren Aufnahme des Kaltwassers wesentlich längere Zeiten, so daß unter Umständen ein kontinuierliches Verfahren zur Rohstoff-Konditionierung nicht mehr durchgeführt werden kann; die Rohstoff-Konditionierung erfolgt dann chargenweise. Für eine Konditionierung des Rohstoffs bei einer Wassertemperatur von 10 °C wird mindestens eine Stunde benötigt, in Abhängigkeit vom Substrat sogar bis zu 15 Stunden.

Der Rohstoff wird sodann vom Tauchwasser abgetrennt und stufenweise in einem Wasch-Kühlprozeß auf Inokuliertemperatur gebracht.

Der Zerkleinerungsgrad des Rohsubstrats spielt ebenfalls eine wichtige Rolle. Zum einem läßt sich durch die Vergrößerung der Oberfläche sowie durch das optimal eingestellte Verhältnis von fester zu flüssiger Phase die Verweilzeit des zerkleinerten Rohstoffs im erhitzten Tauchwasser auf maximal 5 Minuten reduzieren, zum anderen kann das Substrat nach dem Konditionieren mittels Walzenpressen zu handhabbaren "Kuchen" verfestigt werden, wobei dann diese Kuchen zunächst aufgerissen und mit Pilzbrutsubstanz beimpft, wieder zu einer für das spätere Myzel- und Fruchtkörperwachstum optimalen Dichte verpreßt werden können. Der Zerkleinerungsgrad, ausgedrückt durch die mittlere Korngröße bzw. Faserlänge, ist selbstverständlich abhängig von der Art des Rohstoffs und kann von einem Fachmann, insbesondere auch wegen der kurzen Konditionierungszeiten, über ein Chargenscreening leicht optimiert werden. Für faseriges Substrat liegen die optimalen mittleren Faserlängen im Bereich zwischen 5 bis 10 mm bei einer Gleichmäßigkeitszahl nach DIN 66145 von 1,5 bis 2,5.

Für Getreidestroh liegt das Optimum der mittleren Faserlänge bei 8 mm bei einer Gleichmäßigkeitszahl von 2,1 bis 2,2.

Während des Konditionierens sollte sich das Massenverhältnis von zerkleinertem Substrat zu Tauchwasser im Bereich von 1:15 bis 1:25, vorzugsweise im Bereich von 1:18 bis 1:20 bewegen. Nach dem Abtrennen des Tauchwassers vom Feststoff, vorzugsweise mittels einer Bandfilterpresse, wird es zusammen mit dem Waschwasser einem Entkeimungsprozeß, vorzugsweise einer Filtration, unterworfen und kann dann wieder in den Konditionierungsprozeß eingespeist werden. Das Warmspülen des Substrats unter Verzicht auf die Vorfermentation hat eine drastische Minderung des Energiebedarfs in Vergleich zum Stand der Technik zur Folge. Die pro Tonne Substrat aufzubringende Energie beträgt ca. 43 KW Stunden (Vorfermentation: 260 KW Stunden).

Die äußerst kurze Konditionierungzeit von nur 1 bis 3 Minuten im Vergleich mit dem Zeitaufwand für die vegetative Phase erlaubt es, die Konditionierung der gesamten Substratmenge, die für einen Kultivierungszyklus in Massenproduktion benötigt wird, entweder in mehren Teilchargen hintereinander vorzunehmen oder in einer kontinuierlichen Prozeßführung so, daß jeweils gerade soviel konditioniertes Substrat am Ausgang der Konditionierungsvorrichtung entnommen wird, wie am Eingang Rohsubstrat zugeführt wird. Das so konditionierte Substrat wird walzengepreßt, gewaschen, nach Aufreißen auf eine Dichte von ca. 0,2 t/m$^3$ mit Pilzbrutsubstanz innig vermischt, bis es mindestens einen Mischgrad von 0,95 aufweist und sodann in rechteckigen, stapelbaren, festen, flachen Behältern auf eine Dichte von 0,45 t/m$^3$ verdichtet. Diese Behälter können sodann nacheinander in die Anwachshallen eingebracht werden. Das Koniditionieren und Inokulieren im kontinuierlichen oder Konti-Betrieb ermöglicht eine enorme Einsparung an Platz für Fermentations- und Inokulationsräume im Vergleich zum Stand der Technik.

Ein weiterer Vorteil des erfindungsgemäßen Verfahrens liegt in der Verwendung von stapelbaren Kulturkästen, die vorzugsweise aus einem recyclingfähigen Material hergestellt sind und vorzugsweise flach und quaderförmig und mit durchbrochenen Böden und Wänden nach oben offen ausgebildet sind, so daß sie leicht von Hand und/oder mechanisch gefüllt und entleert werden können und leicht zu reinigen sind. Zusätzlich können sie vorzugsweise über Normmaße verfügen, so daß mehrere Behälter zusammen mittels verfahrbarer Paletten leicht transportierbar sind. Solche Behälter können mittels nur an ihrer Ober- und/oder Unterseite an den Ecken überstehender Stege so gestaltet werden, daß sich jeweils zwischen freier Oberseite des einen und durchbrochener Unterseite des darüber gestapelten Behälters ein Spalt ausbildet, durch den Luft hindurchströmen kann. Darüber hinaus ist es aber auch möglich, die Kästen während der vegetativen Wachstumsphase eng nebeneinander zu stapeln, so daß ein Raumnutzungsgrad von mindestens 0,8 ohne weiteres erreicht werden kann. Die nach dem Stand der Technik verwendeten Foliensäcke weisen an dem peripheren Bereich, wo sie nicht aneinander stoßen, erhebliche Toträume auf. Darüber hinaus können sich infolge mangelnder Durchlüftung im Inneren der mit über 0,4 m Durchmesser mehr oder weniger kompakt gefüllten Säcke Temperaturen ausbilden, die eine Kühlung erforderlich machen. Ohne Kühlung könnte sich sonst zwischen Sackmitte und Außenhaut eine Temperaturdifferenz von ca. 22°C ausbilden. Im Inneren der Foliensäcke hätte dies ein Absterben des Myzels zur Folge. Da der Stoffwechsel im Inneren von der Menge der inokulierten Pilzbrutsubstanz abhängt, können Brutgaben über 2% bei einem Verfahren nach dem Stand der Technik unwirtschaftlich werden. Aus diesem Grund dürfen die Foliensäcke in der vegetativen Wachstumsphase nicht zu dicht nebeneinander gestapelt werden.

Anstelle der Anwachshallen werden vorzugsweise Klimakammern verwendet, die in ihrer Dimensionierung optimal auf die Dimensionen der auf den fahrbaren Paletten gestapelten Behälter ausgerichtet sind und zwar so, daß im Falle von vegetativen Klimakammern zwischen den Behältern, den Kammerwänden und der Decke eine Abstand von nicht mehr als 20 cm bestehen bleibt. Der Abstand der einzelnen Behälterstapel voneinander kann dabei so klein wie möglich gehalten werden, z.B. weisen die verdichteten Substrate in übereinander gestapelten Behältern pro Behälter vorzugsweise jeweils einen Abstand von 2-3 cm voneinander auf, wobei die einzelnen Behälterstapel einen seitlichen Abstand von ca. 2 cm und an den

EP 0 483 655 A2

Stirnseiten von ca. 1 cm aufweisen. Darüber hinaus lassen sich diese containerartigen Klimakammern, die vorzugsweise mobil ausgelegt sein können, mit Normmaßen nach Art eines Baukastenprinzips stirnseitig miteinander hermetisch verkuppeln, so daß die Größe der als Anwuchshallen dienenden, miteinander kombinierten Klimakammern optimal auf den erforderlichen Durchsatz und ebenso auf das zur Verfügung stehende Raumangebot ausgerichtet werden kann. Die einzelnen Kammern weisen Wände mit einer hohen Wärmedämmung (K-Wert im Bereich von 0,2-0,35) auf, die aufgrund ihrer glatten Oberfläche leicht zu reinigen sind.

Die kombinierten Klimakammern sind an ihren frei endenden Stirnseiten mit Zu- und Abluftleitungen versehen. In der Zuluftleitung wird mittels Flächenfilter Druckluft erzeugt und zwar so, daß in Längsrichtung der kombinierten Klimakammern angenähert eine Kolbenströmung aufrecht erhalten wird. Das Klima in den Kammern selbst wird nach den bewährten Methoden der Klimatechnik gemäß dem Stand der Technik erzeugt und geregelt.

Nach Beendigung der vegetativen Wachstumsphase werden die mit Behälterstapeln beladenen, verfahrbaren Paletten in Klimakammern für generatives Wachstum verbracht.

In der generativen Wachstumsphase werden erfindungsgemäß die gestapelten Behälter seitlich, wo die Fruchtkörper herauswachsen, nur mit einem so großen Abstand beaufschlagt, daß die sich einander gegenüberstehenden Pilze gerade noch ungehindert aufeinander zuwachsen können. Der Abstand der jeweiligen Stapel wird sich naturgemäß nach der maximalen Größe der verwendeten Pilzart richten. Für Pleurotus sp. beträgt der Abstand vorzugsweise 20 cm. Ein gleichmäßiges Größenwachstum der Fruchtkörper, insbesondere vorteilhaft bei Zuhilfenahme mechanischer Erntevorrichtungen, kann damit erreicht werden. Demgemäß können aber in einer generativen Klimakammer weniger Behälterstapel nebeneinander stehen, was zur Folge hat, daß mehr Einelkammern hintereinander gekoppelt werden müssen. Selbstverständlich sind für vegetative und generative Klimakammern gesonderte Zu- und Abluftleitungen vorgesehen und auch die Klimatisierung ist spezifisch auf die jeweilige Wachstumsphase eingestellt. Die Raumeinsparung und die mit der Klimaregelung verbundene Energieersparnis gegenüber dem Stand der Technik liegen auf der Hand.

Infolge des gleichmäßigen Fruchtkörperansatzes kann die Ernte leicht mechanisiert werden, z.B. unter Verwendung oszillierender Schneidklingen und/oder eines umlaufenden Schneiddrahtes.

In dem Falle, daß die Pilze von Hand geerntet werden, sind Vorrichtungen vorgesehen, die dafür Sorge tragen, daß ständig von den Atemwegen des Erntepersonals zu den abzuerntenden Pilzen hin ein Luftstrom aufrechterhalten wird, über den die beim Ernten aufgewirbelten Sporen abgesaugt werden können. Auf diese Weise werden allergetische Erkrankungen, die mit den Pilzen korreliert sind, insbesondere die Pleurotus-Sporenallergie, größtmöglich vermieden. Vorzugsweise sind auch Vorrichtungen direkt im Anschluß an die Klimakammern vorgesehen, um die Pilze unmittelbar nach der Aberntung vom Substrat kühlen zu können.

Im Folgenden wird das Kultivierungsverfahren am Beispiel von Pleurotus Spezies näher beschrieben.

Mindestens 3 Monate altes Getreidestroh, dessen Feuchtigkeit 15% nicht überschreiten sollte und das möglichst frei von Pflanzenschutzmittelrückständen sein sollte wird in einem Trommel- bzw. Scheibenradhäcksler nach dem Schneidverfahren und anschließend in einer Hammermühle zerkleinert. Die Körnung des Haufwerks wird so eingestellt, daß die Substratpartikel eine mittlere Faserlänge von 8 mm aufweisen mit einer Gleichmäßigkeitszahl nach DIN 66145 von 2 bis 2,2.

Nach erfolgter Zerkleinerung wird der Rohstoff in Wasser suspendiert, wobei das Massenverhältnis von Substrat zu Wasser sich zwischen 1:18 und 1:20 bewegen soll. Die Suspension wird sodann auf ca. 65°C erwärmt und bis 5 Minuten bei dieser Temperatur belassen. Danach wird das Tauchwasser mittels einer Bandfilterpresse vom konditionierten Substrat abgetrennt, dieses mit einer Walzenpresse zu einem Kuchen ausgepreßt, der 1 bis 2 Mal mit kaltem Wasser gewaschen wird. Nach dem Pressen soll sich das Verhältnis von zerkleinertem konditionierten Stroh zu Wasser in Abhängigkeit von der Feinheit des zerkleinerten Substrats zwischen 1:1 und 1:7 bewegen. Für Substrat mit Faserlängen von 8mm liegt das Verhältnis vorzugsweise bei 1:2,5 bis 1:4.

Als nächster Schritt folgt die Inokulation des konditionierten Substrats. Dazu wird der gepreßte Substratkuchen mechanisch aufgerissen und mit Brutsubstanz versetzt. Als Pilzbrut dient eine auf Getreide (Hirse, Roggen, Weizen) gezogene Reinkultur. Mittels eines Brutdosierreglers werden 1,5 bis 3 Massenteile Brut auf 100 Teile Substrat zugegeben und anschließend intensiv durchmischt. Der Mischungsgrad soll etwa 0,95 betragen. Die Dichte des inokulierten Substrats beträgt ca. 0,2 $t/m^3$.

Mit einem Volumendosierer wird das inokulierte Substrat in die stapelbaren, offenen Behälter gefüllt und anschließend mit einem Vibrator so verdichtet, daß die Substratdichte bei ca. 0,4 $t/m^3$ liegt.

Die übereinander gestapelten Substratbehälter werden nun in die vegetativen Klimakammern (Anwachskammer) eingeführt, wo die einzelnen Stapel so aneinander stehen, daß sie insgesamt mindestens 80% des gesamten Kammervolumens einnehmen. In der Kammer sollen Temperaturen von 19 bis 28°C, vorteilhafterweise 22°C herrschen bei einer relativen Luftfeuchte von 93 bis 100%. In der Klimakammer muß ein Gaswechsel von stündlich 50 bis 500 $m^3/t$ erzielt werden, wobei der Anteil des gebildeten $CO_2$ in der Kammer Abluft zwischen 1000 und 5000 ppm betragen bzw. der $O_2$-Partialdruck nicht unter 0,2 bar sinken soll.

Nachdem das Substrat völlig mit Myzel durchwachsen ist, je nach Spezies zwischen 12 und 25 Tagen, werden die Substratbehälter in die generativen Klimakammern (Erntekammern) verbracht. Dabei werden die seitlichen Abstände der Stapel auf ca. 20 cm eingestellt, so daß die an den Seiten aus den Behältern wachsenden Fruchtkörper zweier sich gegenüberstehender Stapel jeweils eine Wuchslänge von ca. 10 cm zur Verfügung haben. Der Raumnutzungseffekt wird dadurch von 0,8 auf ca. 0,35 verringert. Das Klima in der Erntekammer besteht aus einer Temperatur von 18 bis 25°C, vorzugsweise 18 bis 20°C und einer relativen Luftfeuchtigkeit von 70 bis 100%, vorzugsweise 80-90% Die spezifische, pro Stunde umgewälzte Luftmenge soll 250 bis 750 $m^3/t$ betragen, wobei sich der $CO_2$-Gehalt auf 500 bis 1000 ppm einpendelt.

Im Folgenden wird anhand einer Tabelle die Einsparung an Energie, Raum und Zeit während der Substratbereitung dem Stand der Technik gegenübergestellt:

## Tabelle

## Energie-, Raum- und Zeitersparnis beim Konditionieren des Substrats im Vergleich zum Stand der Technik

| | Stand der Technik | erfindungsgemäße Lösung | Einsparung in % |
|---|---|---|---|
| Energiebedarf[*] $Q_{th+mech}$ [kWh/t Substrat] | 260 | 43 | 83 |
| Flächenbedarf[**] $m^2/t$ | 0,25 | 0,01 | 96 |
| Zeitaufwand | 2-5 d | 1-3 min | 99,9 |

[*] einschließlich Zerkleinerung

[**] bei einer durchgängigen Höhe von 2,5 m

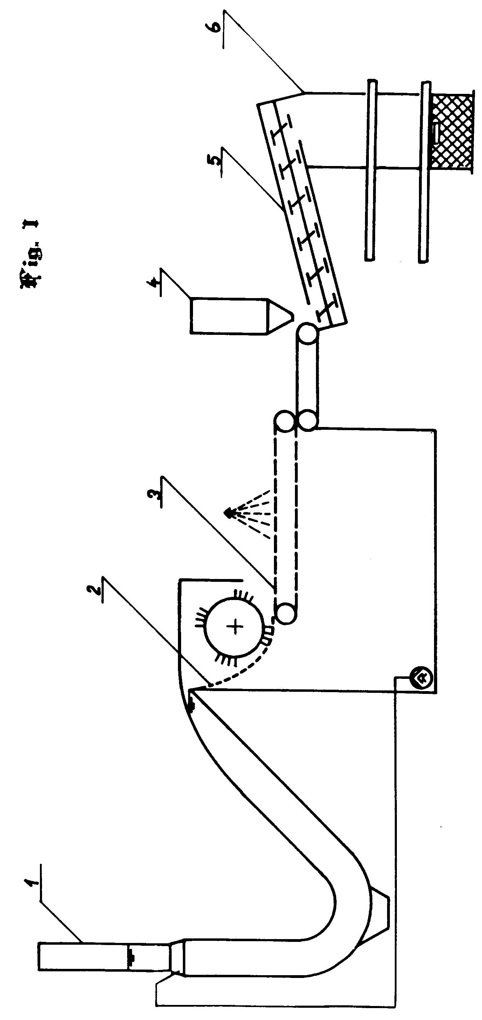

Figur 1 zeigt eine Vorrichtung für die Substratbereitung nach dem Konti-Verfahren.

Der von einer (nicht eingezeichneten) Schneid- oder Hammermühle zerkleinerte Rohstoff wird in das Tauchbad 1 eingebracht. Der im Tauchwasser suspendierte zerkleinerte Rohstoff wird über Bogensieb 2 vorgefiltert und auf eine Bandsiebapparatur mit Wäscher 3 weitergeführt. Der Wäscher kühlt durch Aufsprühen von Wasser das zerkleinerte Substrat auf Inokuliertemperatur. Nach Pressen des zerkleinerten Substrats mittels (nicht wiedergegebener) Bandfilter- und Walzenpressen wird diesem über die Dosiervorrichtung (Brutdosierregler) 4, an die vorzugsweise eine Bandwaage angeschlossen ist, Pilzbrutsubstanz zugesetzt. Der sich anschließende Mischapparat 5 bringt das Substrat und die Pilzbrutsubtanz auf den erforderlichen Mischgrad und verdichtet das nun mit Pilzbrutsubstanz inokulierte Substrat. In der an einen Volumendosierer angeschlossenen Behälterfüllvorrichtung 6 wird das verdichtete Substrat nach Befüllen der Behälter nochmals auf die endgültige optimale Substratdichte verpreßt.

Die Vorteile des vorliegenden Verfahrens liegen auf der Hand. Nicht nur wegen der damit verbundenen Zeit- und Energieeinsparung gegenüber dem Stand der Technik, sondern auch in bezug auf die Anwendung selbst. Insbesondere dort, wo äußere Umstände eine wechselnde Nachfrage nach unterschiedlichen Ausbeuten der anfallenden Kulturpilze bzw. Zwischenprodukte bedingen, bietet das koninuierliche Konditionierungsverfahren (Konti-Verfahren) in Verbindung mit dem baukastenartigen Aufbau der Klimakammern Möglichkeiten, sich schnell otpimal an die jeweiligen Erfordernisse anzupassen. Darüber hinaus sind die containerartigen Klimakammern noch mobil ausgebildet, so daß wegen der ebenfalls mobil und auf kleinstem Raum ausbildbaren Konditionierungsvorrichtung der Produktionsort beliebig gewechselt werden kann. Dies ist insbesondere bei einem Einsatz in der Umwelttechnik ein nicht zu unterschätzender Vorteil.

Nach Beendigung der vegetativen Wachstumsphase kann zum Beispiel das dann mit Myzel durchwachsene Substrat zur Entsorgung von mit Xenobiotica verseuchtem Erdreich eingesetzt werden, da das Myzel solche Stoffe teils direkt verstoffwechseln kann, teils über Ausscheidungsprodukte zersetzen oder zumindest zu ungiftigen Substanzen abbauen kann. Eine Anpassung der Anlage an die Erfordernisse sowohl in Hinblick auf Kapazität als auch Spezifität, noch dazu in situ, ist mit dem vorliegenden Verfahren möglich.

Selbstverständlich sind auch noch andere Anwendungsgebiete, wie zum Beispiel auf dem Nahrungsmittelsektor, im Pflanzenschutz oder der Tierproduktion denkbar. Aus der gebildeten Biomasse oder deren Ausscheidungsprodukten können zum Beispiel auch pharmazeutisch wirksame Substanzen isoliert werden, deren Herstellung über die Isolierung aus kultivierten saprophytischen Pilzen einer chemischen Synthese in Hinblick auf Rentabilität oft vorzuziehen ist.

## Patentansprüche

1.  Verfahren zum Kultivieren saprophytisch lebender Pilze auf im wesentlich aus pflanzlichen, lignozellulosehaltigen Produkten bestehendem Substrat, wobei das Substrat vor der Inokulation mit Pilzbrutsubstanz zunächst konditioniert, nach der Inokulation in geeigneten, stapelbaren Behältern verdichtet, in der vegetativen Wachstumsphase mit Luft durchströmt und sodann in der generativen Wachstumsphase der Raumnutzungsgrad gesenkt wird, dadurch gekennzeichnet, daß
    - das Substrat während der Konditionierung in Wasser von 50 bis 70 °C, vorzugsweise 65°C, über einen Zeitraum im Bereich von 10 Sekunden bis 5 Minuten, vorzugsweise 1 bis 3 Minuten, eingetaucht wird,
    - das entwässerte, zerkleinerte Substrat nach der Inokulation mit Pilzbrutsubstanz in geeigneten, flachen, stapelbaren, an den Seiten und am Boden durchbrochenen, nach oben offenen, festen Behältern auf 0,35 bis 0,5 t/m$^3$ vorzugsweise 0,4 t/m$^3$, verdichtet wird, wobei zur Vermeidung von Substratüberhitzung in der anschließenden vegetativen Wachstumsphase die Höhe der Behälter so bemessen ist, daß mittels Wärmeaustausch zwischen Behälterinhalt und diesen Behälter überstreichender Luft eine Temperatur von 30°C im Zentrum des mit Substrat beschickten Behälters nicht überschritten wird,
    - bei einem Raumnutzunsgrad von mindestens 0,8 in der vegetativen Wachstumsphase 50 bis 500 m$^3$, vorzugsweise 100 bis 300 m$^3$ Luft pro Stunde und Tonne Substrat durch den Raum mit den mit Substrat beschickten, gestapelten Behältern strömen gelassen wird,
    - der Raumnutzungsgrad in der generativen Wachstumsphase unter seitlicher Abstandsvergrößerung der Stapel auf 0,2 bis 0,5, vorzugsweise 0,35 bei gleichbleibender Luftdurchströmung, aber verminderten $CO_2$-Partialdruck gesenkt wird, so daß die sich bildenden Fruchtkörper an den seitlichen Behälteroberflächen ungehindert in den Raum wachsen können, und
    - bei der manuellen Ernte ständig ein Luftstrom in Richtung von den Atemwegen des Erntepersonals auf die abzuerntenden Pilze aufrechterhalten wird.

2.  Verfahren nach Anspruch 1, dadurch gekennzeichnet, daß das Substrat vor der Konditionierung auf eine Korngröße bzw. Faserlänge von 5 bis 10 mm, im Falle einer Faser vorzugsweise auf 8 mm, mit einer Gleichmäßigkeitszahl nach DIN 66145 von 1,5 bis 2,5, vorzugsweise < = 2 zerkleinert wird.

3.  Verfahren nach Anspruch 1 und 2, dadurch gekennzeichnet, daß das zerkleinerte Substrat zum Konditionieren in Wasser in einem Massenverhältnis von 1:15 bis 1:25, vorzugsweise 1:18 bis 1:20, suspendiert wird.

4.  Verfahren nach den Ansprüchen 1-3, dadurch gekennzeichnet, daß die gesamte für einen Kultivierungszyklus erforderliche Substratmenge in einem kontinuierlich ablaufenden Prozeß konditioniert wird.

**5.** Verfahren nach Anspruch 1 bis 4, dadurch gekennzeichnet, daß restliches Wasser nach der Konditionierung vom festen Substrat abgepreßt wird, dieses mit kalten Wasser mindestens einmal gewaschen wird und anschließend das Massenverhältnis von Substrat/Wasser auf 1:2 bis vorzugsweise 1:4 eingestellt wird.

**6.** Verfahren nach Anspruch 5, dadurch gekennzeichnet, daß das abgepreßte Wasser und das Waschwasser zur Entfernung der Sporen von Schadpilzen einer Teilentkeimung vorzugsweise über eine Filtration unterzogen werden und dann wieder in den Konditionierungsprozeß eingespeist werden.

**7.** Verfahren nach den Ansprüchen 1 bis 5, dadurch gekennzeichnet, daß die Mischung von Substrat und Inokulat mindestens einen Mischgrad von 0,95 aufweist.

**8.** Verfahren nach Anspruch 1, dadurch gekennzeichnet, daß die mit inokuliertem Substrat gefüllten, stapelbaren Behälter vor der vegetativen Wachstumsphase auf verfahrbaren Paletten gestapelt werden.

**9.** Verfahren nach den Ansprüchen 1 und 8, dadurch gekennzeichnet, daß die mit den mit inokuliertem Substrat gefüllten stapelbaren Behältern bestückten verfahrbaren Paletten jeweils für die vegetative und generative Wachstumsphase in mobile, genormte, untereinander hermetisch verkoppelbare Klimakammern verbracht werden.

**10.** Verwendung des nach der vegetativen Wachstumsphase mit Myzel bewachsenen Substrats gemäß Anspruch 1 zur Entsorgung von mit Xenobiotica kontaminiertem Erdreich.

Fig. 1

EP 0 483 655 A2